# EUROPEAN PATENT SPECIFICATION

(11) **EP 3 527 513 B1**
(45) Date of publication and mention of the grant of the patent: **11.11.2020**
(21) Application number: 18156878.3
(22) Date of filing: 15.02.2018
(51) Int. Cl.: B65G 63/00, G01N 33/00, G08B 21/14

(54) **METHOD AND SYSTEM TO RELEASE A CONFINED STOWAGE UNIT FOR UNLOADING OR INSPECTION**
VERFAHREN UND SYSTEM ZUR FREIGABE EINER GESCHLOSSENEN FRACHTEINHEIT ZUM ENTLADEN ODER INSPIZIEREN
PROCÉDÉ ET SYSTÈME POUR LIBÉRER UNE UNITÉ DE CARGAISON FERMÉE POUR LE DÉCHARGEMENT OU L'INSPECTION

(43) Date of publication of application: 21.08.2019
(73) Proprietor: Atmosafe BVBA, 2040 Antwerpen (BE)
(72) Inventor: Boodts, Peter, 2800 Mechelen (BE); Bonneux, Tom, 2530 Boechout (BE); Hertog, Joachim, 2060 Antwerpen (BE)
(74) Representative: IP HILLS NV

(56) References cited:
- WO-A2-2006/086507
- CN-U- 206 088 400
- US-A- 4 580 440

## Description

### Field of the Invention

The present invention generally relates to unloading or physically inspecting confined stowage units, more particularly to evaluating the safety of people involved in such unloading or physical inspection process by analysing the air or atmosphere in such confined stowage unit for intoxication and asphyxiation before releasing it for unloading or physical inspection.

### Background of the Invention

A confined stowage unit, for instance a shipping container or a vessel's cargo hold, is used to transport or store bulk commodities, e.g. grains, fertilizers, etc., semi-finished materials, e.g. steel plates, polymers, etc., finished products, e.g. shoes, toys, etc., or other goods. The confined stowage unit remains closed during the entire voyage which may take several weeks and will be opened upon arrival for the first time by workers, dockworkers or customs to inspect or unload the cargo. Opening or physically entering the confined stowage unit is potentially dangerous for people because of the accumulation of hazardous gases and vapours inside the confined stowage unit.

International patent application WO 2006/116809 A1 entitled "An Improved Residual Gas Removal Method" describes the risk for persons that access shipping containers to come in contact with toxic gases and the importance of venting shipping containers to reduce the risk for persons that access containers on page 1, lines 28-36. WO 2006/116809 A1 further describes an iterative process for removing residual gas from a shipping container, maintaining a sub-atmospheric pressure.

United States Patent Application US 2010/0112680 A1, entitled "Indicator System for Determining Analyte Concentration" also mentions the risk of human exposure to toxic gases when entering a closed or partially closed environment in paragraph [0004]. US 2010/0112680 A1 further describes a method for developing an exposure history of a load to target molecules using an indicator system inside a container that records at regular times molecular diffusion of these target molecules. The technique in particular envisages food, beverage and pharmaceutical loads.

WO2006/086507 discloses an analyser measuring concentrations of an inventory of gases and a generic sensor for measuring the concentration of volatile organic compounds, said analyser and said sensor being used on air samples taken from a cargo container.

In order to determine if a confined stowage unit can be released safely for unloading or physical inspection, an air sample taken of the closed confined stowage unit can be analysed for risks of intoxication and asphyxiation upon arrival, before opening of the confined stowage unit takes place. Typically, such air sample analysis results in an inventory of up to 50 potentially dangerous gases per confined stowage unit, the so-called risk inventory listing the potentially dangerous gases and their respective concentrations in the air sample. The air sample analyses take place at a central location where multiple confined stowage units arrive, for instance the dock where the shipping containers are unshipped. Because of the high volume of stowage units present at such central location, the cost per analysis goes down, justifying the use of expensive and complex air sample analysers. The cost of such an air sample analyser ranges from 50.000 EUR upward.

The risk inventory is used to generate a report, the so-called risk evaluation, containing a list of gases/vapours the concentrations of which exceed the acceptable safety limits, e.g. Threshold Limit Values or TLVs, and a recommended aeration time, e.g. 2 hours, for the confined stowage unit before unloading, manipulation or physical inspection. After aeration, a secondary analysis or so-called verification is required to verify whether the concentrations of all gases/vapours are now all below their TLVs. If not, the aeration time should be extended until this is the case. Thereafter, during the discharging and/or manipulation process, it may be necessary to further verify if the concentrations of gases/vapours stay below their TLVs. Indeed, during entry of the stowage unit and/or during manipulation of the goods, concentrations of gases/vapours may rise again due to accelerated evaporation of toxic compounds at higher temperature or the release of so-called gas pockets, which are isolated areas between the goods with higher concentrations of certain gases/vapours. Hence, the continued verification or so-called monitoring of the actual concentrations of gases/vapours during the manipulation and/or discharging process may be necessary to guarantee safe working conditions for the people involved in these processes.

After the risk inventory at a centralized location, the unloading or physical inspection of the confined stowage unit often is decentralized, e.g. near the store, shop or factory where the confined stowage unit is transported to from the centralized location. Consequently, aeration, verification and monitoring of the air in the confined stowage unit must also be organized decentralized to ensure workers at the decentralized location are protected from exposure to dangerous, chemical compounds.

A decentralized air sample analysis however is time consuming, expensive and cumbersome as it involves either an air sample analyser or a series of specific gas measurements, one for each of the toxic gases mentioned in the risk evaluation. An air sample analyser is an expensive, bulky and complex system, difficult to transport to or to be available at each decentralized location where confined stowage units are released. A series of specific gas measurements on the other hand adds up to a considerable cost because of disposables and it is time consuming.

Furthermore, subsequent monitoring of the concentrations of the present toxic gases during inspection or manipulation of the goods is subject to the same complexity and has similar disadvantages.

It is therefore an objective of the present invention to disclose a method and system for releasing a confined stowage unit for unloading or physical inspection at a decentralized location that is less cumbersome, less time consuming and less expensive without impacting the safety of workers.

### Summary of the Invention

According to the present invention, the above defined objective is realised by the method to release a confined stowage unit for unloading or physical inspection as defined by claim 1, the method comprising:
A. an air sample analyser at a central location with multiple confined stowage units analysing a first air sample of the confined stowage unit and determining an inventory of gases present in the first air sample with their gas concentrations;
B. a first generic sensor measuring at the central location a first generic sensor value for the first air sample, a generic sensor being a sensor providing a read-out value, named the generic sensor value, that is indicative for the presence of one or several compounds that are not necessarily dangerous, and that can be related to the concentration or evolution of the concentration of one or several toxic compounds in said inventory;
C. a processor determining a safe generic sensor value from the gas concentrations in the inventory and the first generic sensor value, and producing a risk evaluation report for the confined stowage unit, the risk evaluation report comprising an aeration recommendation and the safe generic sensor value;
D. a second generic sensor similar to the first generic sensor measuring at a decentralized location whereto the confined stowage unit is transported and where the confined stowage unit has been aired according to the aeration recommendation, a second generic sensor value for a second air sample of the confined stowage unit;
E. comparing the second generic sensor value with the safe generic sensor value; and
F. releasing the confined stowage unit for unloading or physical inspection if the second generic sensor value is below the safe generic sensor value.

Thus, according to the invention, a centralized air sample analysis solves the disadvantage of high costs for obtaining a risk inventory for the confined stowage unit, e.g. the shipping container, as the high cost for an air sample analyser can be shared over a high amount of analyses that take place at the central location. In addition to a centralized air sample analysis, a generic sensor value is also determined centrally. The generic sensor value reading is produced using a generic, low cost sensor like for instance a Volatile Organic Compounds (VOC) sensor, e.g. a photo-ionization detector, an electrochemical sensor, a diode/heated pentode or the like. The generic sensor provides a read-out value, named the generic sensor value, that is indicative for the presence of one compound or several compounds that is/are not necessarily dangerous. The read-out value can be related to the concentration or evolution of the concentration of one or several toxic compounds in the inventory, for instance through a table or formula. This generic sensor value can then be used as a typical value for the composition of the atmosphere in the confined stowage unit. Using the initial air sample analysis together with this generic sensor value, a safe generic sensor value is calculated depending on the concentrations and TLVs of the detected dangerous compounds. This safe generic sensor value is mentioned in the risk evaluation report accompanying the confined stowage unit, together with a recommended aeration time, i.e. the duration of the time interval during which that confined stowage unit should be aired before it is accessed by workers. Note that this safe generic sensor value does not have to be a direct readout of the compound which is found above its TLV. Any response of the generic sensor can be used. Because of this technique, the decentralized air sample analysis can be reduced to a second generic sensor value reading that is compared with the safe generic sensor value, mentioned in the risk evaluation report accompanying the confined stowage unit. When this second generic sensor value is below the safe generic sensor value, the atmosphere inside the confined stowage unit is safe for entry and the cargo is released for unloading or physical inspection. Otherwise, the confined stowage unit is further aired before a new generic sensor value measurement takes place. Similarly, measurement of further generic sensor value(s) and comparison of such further generic sensor value(s) to the safe generic sensor value can be used to continuously monitor the level of concentrations of gases/vapours in the stowage unit during discharge or manipulation of the goods.

The present invention avoids the necessity of a complete decentralized air sample analysis with bulky expensive air analysers or a costly series of specific gas measurements. Compared to existing solutions, the method is cost efficient through the use of a generic sensor value determined by using a small, low-cost sensor like a VOC sensor, an electrochemical sensor, a diode/heated pentode, or the like. Such sensor has a typical cost of 800 à 8000 EUR. Sensors of this type have a response time of less than 30 seconds and are fool proof. No expertise is needed to handle these devices. The invention consequently facilitates a faster and simpler, decentralized verification of a confined stowage unit before release for unloading and physical inspection.

Optionally, as defined by claim 2, the method according to the invention further comprises iteratively repeating the steps D and E until the second generic sensor value is below the safe generic sensor value and supplementary airing the confined stowage unit for a predetermined time between subsequent iterations of the steps D and E as long as the second generic sensor value is above the safe generic sensor value.

Indeed, as long as the second generic sensor value measured at the decentralized location is above the safe generic sensor value mentioned in the risk evaluation report, it must be assumed that one or more potentially dangerous compounds may be present in a concentration that is too high for safe release of the confined stowage unit. Additional aeration of the confined stowage unit is applied for a predetermined time interval, e.g. 30 minutes. Thereafter, a new readout of the second generic sensor takes place and the new second generic sensor value is again compared with the safe generic sensor value mentioned in the risk evaluation report accompanying the confined stowage unit. This process of additional aeration, generic sensor value measurement, and comparison of the generic sensor value with safe generic sensor value is repeated iteratively until the generic sensor value measured at the decentralized location is below the safe generic sensor value. Then, the confined stowage unit is declared safe for unloading or physical inspection.

Further optionally, as defined by claim 3, the method according to the present invention comprises iteratively repeating the steps D and E at any time during unloading or physical inspection for the purpose of monitoring the gas concentrations and evaluating the safety of workers involved in the unloading or physical inspection.

Indeed, the presence of a fast, simple, inexpensive generic sensor at the decentralized location enables easy monitoring of concentrations of potentially toxic gases during the unloading or physical inspection of the confined stowage unit. As such, changing conditions can be detected fast, and workers involved in the unloading or physical inspection can be warned timely should concentrations of one or more potentially toxic gases again rise above their TLVs. The second generic sensor may be placed in the zone where workers involved in the inspection or discharging process are operational. The second generic sensor may for instance be attached magnetically to a wall of the confined stowage unit, may be attached mechanically to a hook in the confined stowage unit, or may be attached to the workers.

Optionally, as defined by claim 4, the method according to the present invention comprises generating a visual or audible alarm if the second generic sensor value is above the safe generic sensor value.

In particular when gas concentrations are continuously monitored during the unloading or physical inspection through repeated generic sensor measurements, it is advantageous that an audible or visible alarm is generated by the second generic sensor as soon as its measurements exceeds the safe generic sensor value. This way, workers are warned of an unsafe condition without having to inspect the generic second sensor themselves.

As defined by claim 5, the first generic sensor and the second generic sensor may be VOC sensors or photoionization detectors (PID).

Alternatively, as defined by claim 6, the first generic sensor and the second generic sensor may be electrochemical sensors.

According to yet another alternative, defined by claim 7, the first generic sensor and the second generic sensor may be diode/heated pentode sensors.

According to a further alternative, defined by claim 8, the first generic sensor and the second generic sensor may be infrared sensors.

According to a further alternative, defined by claim 9, the first generic sensor and the second generic sensor may be catalytic sensors.

The skilled person will appreciate that the above list of examples of first and second generic sensors is non-exhaustive. Any sensor that is portable, relatively inexpensive in comparison with an air sample analyser, and which produces a read-out that is indicative for the presence of one or several compounds that are not necessarily the detected dangerous compounds, can be used. It is however important that a similar generic sensor is used at the centralized location where the air sample analysis takes place and where the risk evaluation report is produced, and at the decentralized location where the verification takes place before release of the confined stowage unit. In other words, if a VOC sensor is used at the centralized location, a VOC sensor must be used at the decentralized location; if a diode/heated pentode sensor is used at the centralized location, a diode/heated pentode sensor must be used at the decentralized location; etc. It is also important that the generic sensor reading can be related somehow to the concentration of the toxic compounds detected in the air sample at the centralized location, or at least that there is some knowledge on the evolution of the generic sensor reading versus the evolution of the concentration of the toxic compound(s) detected in the air sample. This relation may be expressed in a formula or in tables known by the processor that determines the safe generic sensor value from the toxic gas concentrations and the generic sensor reading at the centralized location.

In preferred embodiments of the method according to the invention, as defined by claim 10, no further analysis of the second air sample is executed at the decentralized location except the second generic sensor value measurement.

Indeed, the value of the present invention lies in the fact that no air sample analysis is done at the location where the confined stowage unit is released for unloading or inspection. Consequently, no bulky and expensive air sample analyser must be present and no expert technicians able to configure such air sample analyser must be de-located. This simplifies the process of releasing confined stowage units for unloading or physical inspection substantially, and substantially adds to the effectiveness and cost gain of organizing the release of confined stowage units, in particular in countries or areas where the release is organized decentralized for practical or legal reasons.

In embodiments of the method according to the present invention, defined by claim 11, the risk evaluation report comprises a machine-readable code wherein the aeration recommendation and/or the safe generic sensor value are encoded.

Thus, the method is made less error-prone or more fool proof by encoding the recommended aeration time and/or the safe generic sensor value, both calculated by the processor, in a machine-readable code. The machine-readable code can be a barcode, a Quick Response (QR) code, a Radio Frequency Identification (RFID) code, or any alternative machine-readable code. The code is embedded in the risk evaluation report, i.e. a paper document, a tag or a digital document. In case of a paper or analogue document, the document may accompany the confined stowage unit when transported from the central location to the decentralized location. In case of a tag, the tag may be attached to the confined stowage unit. This brings the advantage that the confined stowage unit itself carries its aeration time and/or safe generic sensor value. A digital version of the risk evaluation report can be sent by e-mail, sms, or the like over the Internet or another communication network to a person at the decentralized location, or directly to the second generic sensor if the second generic sensor forms part of a sensor unit with network connectivity and ability to receive/interpret digital messages. The latter situation brings the advantage that the safe generic sensor value is programmed in the second generic sensor without any human intervention or assistance as a result of which the monitoring of the confined stowage unit is made completely fool proof.

In addition to a method as defined by claim 1, the present invention also concerns a corresponding system for releasing a confined stowage unit for unloading or physical inspection as defined by claim 12, the system comprising:
A. an air sample analyser adapted to analyse a first air sample of the confined stowage unit at a central location with multiple confined stowage units, and configured to determine an inventory of gases present in the first air sample with their gas concentrations;
B. a first generic sensor adapted to measure at the central location a first generic sensor value for the first air sample, a generic sensor being a sensor providing a read-out value, named the generic sensor value, that is indicative for the presence of one or several compounds that are not necessarily dangerous, and that can be related to the concentration or evolution of the concentration of one or several toxic compounds in said inventory;
C. a processor configured to determine a safe generic sensor value from the gas concentrations in the inventory and the first generic sensor value, and configured to produce a risk evaluation report for the confined stowage unit, the risk evaluation report comprising an aeration recommendation and the safe generic sensor value;
D. a second generic sensor similar to the first generic sensor, adapted to measure at a decentralized location whereto the confined stowage unit is transported and where the confined stowage unit has been aired according to the aeration recommendation, a second generic sensor value for a second air sample of the confined stowage unit for comparison with the safe generic sensor value, and release of the confined stowage unit for unloading or physical inspection if the second generic sensor value is below the safe generic sensor value.

In embodiments of the system according to the present invention, defined by claim 13:
- the processor is configured to generate a machine-readable code wherein the aeration recommendation and/or the safe generic sensor value are encoded, and to embed the machine-readable code in the risk evaluation report; and
- the second generic sensor comprises or connects with a code reader adapted to read the machine-readable code and to extract therefrom the aeration recommendation and/or the safe generic sensor value.

As already mentioned above, the processor that calculates the safe generic sensor value and the recommended aeration time for a confined stowage unit, in advanced embodiments of the invention encodes these two values in a machine-readable code, for instance a barcode, QR code or RFID code. Such code may then be tagged to the confined stowage unit, may be printed on a paper version of the risk evaluation report, or may be sent by e-mail, sms or some other digital signal to a terminal at the decentralized location, for instance a person's smartphone, laptop or the second generic sensor itself if this sensor is equipped with a digital transceiver able to receive and interpret digital messages. The second generic sensor in such embodiments of the invention preferably is equipped with a barcode reader, QR scanner, RFID reader or the like such that it is able to scan a tag attached to the confined stowage unit, a code printed on paper, or a code displayed on the screen of a terminal. The use of a machine-readable code to convey the safe generic sensor value and/or recommended aeration time from the central location to the decentralized location further reduces the risk for human errors.

In embodiments of the system according to the present invention, defined by claim 14:
- the second generic sensor comprises an alarm unit configured to generate a visual or audible alarm if the second generic sensor value is above the safe generic sensor value.

Indeed, the second generic sensor preferably has an integrated alarm unit that automatically generates an audible or visible alarm as soon as its measurements exceeds the safe generic sensor value. The safe generic sensor value may be entered by a person at the decentralized location or may be received from the processor at the central location of the second generic sensor also incorporates a transceiver, able to receive and interpret for instance e-mails, sms messages or other digital messages carrying the safe generic sensor value. Thanks to the audible or visible alarm, it is no longer necessary that a person at the decentralized location regularly inspects the second sensor measurement. The second generic sensor can continuously generate second generic sensor values, compare these values with the safe generic sensor value and generate an alarm when the safe generic sensor value is exceeded in order to create permanent safe working conditions at the decentralized location where the confined stowage unit will be unloaded or entered for inspection.

### Brief Description of the Drawings

Fig. 1 illustrates an embodiment of the system to release a confined stowage unit according to the present invention;
Fig. 2 is a diagram illustrating the steps executed at a centralized location in an embodiment of the method to release a confined stowage unit according to the present invention;
Fig. 3 is a diagram illustrating the steps executed at a decentralized location in an embodiment of the method to release a confined stowage unit according to the present invention; and
Fig. 4 illustrates a suitable computing system 400 for executing the processing steps in embodiments of the method according to the invention.

### Detailed Description of Embodiment(s)

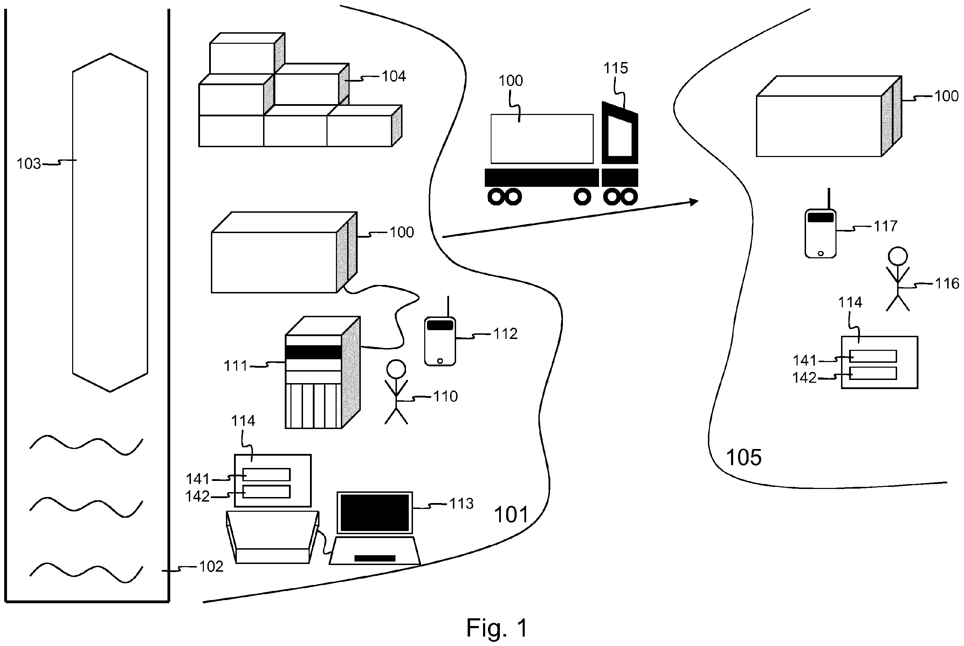

Fig. 1 shows a quay 101 along a shipping dock 102 where a ship 103 has arrived and a large number of shipping containers 100, 104 are unshipped. The shipping containers store bulk commodities like grains, fertilizers, etc., semi-finished goods like polymers, steel plates, etc., and/orfinished products like shoes, clothing, toys, etc. The containers have been travelling for weeks as the ship 103 may be assumed for instance to have travelled from Hong Kong to Antwerp. During the voyage, hazardous, potentially toxic gases and vapours may have accumulated inside one or several containers. Uncontrolled opening of a container by a dockworker or customs for inspection of the goods inside the container may pose the dockworker or customs at risk of being intoxicated. Exceptionally, the risk may even by life-threatening.

In order to analyse the risk of entering or physically inspecting a container 100, a risk analysis will be performed on the container 100 at the central location, i.e. the quay 101 where the containers 100, 104 are unshipped. Thereto, an air sample analyser 111 that is able to detect and identify the presence of tens, i.e. any value in the range from 10 to 100, or even hundreds, i.e. any value in the range from 100 to 1000, of potentially dangerous compounds under control of an expert technician 110 analyses an air sample of the container 100. The air sample analyser 111 determines and identifies the presence of dangerous compounds - typically tens of potentially dangerous compounds are present - as well as the concentration of these identified dangerous compounds in the air sample taken from the container 100. This results in a risk inventory being generated for the container 100. The risk inventory produced for container 100 in Fig. 1 is as follows:

| Risk inventory | | | |
|---|---|---|---|
| Compound | Concentration (ppm) | TLV (ppm) | Exceedance factor (Concentration/TLV) |
| Carbon dioxide | 1278 | 5000 | 0.25 |
| Methane | 2.6 | 1000 | 0.0026 |
| Carbon monoxide | 5.4 | 25 | 0.216 |
| Methanol | 2.7 | 200 | 0.0135 |
| Alfa-pinene | 1.6 | 20 | 0.08 |
| Cyclohexane | 4.2 | 100 | 0.042 |
| Toluene | 6.4 | 20 | 0.32 |
| Benzene | 4.6 | 1 | 4.6 |
| Pentane | 18.4 | 600 | 0.031 |
| 1,2-dichloroethane | 14.6 | 10 | 1.46 |

In the risk inventory, TLV represent the Threshold Limit Values or any acceptable safety limits for the concentration of the various compounds, expressed in parts per million (ppm). In case one or several concentrations exceed the respective TLV(s), the container cannot be released for unloading or physical inspection, but first must be aired. Thereto, the processor 113, coupled to the air sample analyser 111 shall calculate a recommended aeration duration from the risk inventory, i.e. a minimum time interval during which the container 100 must be aired in order to reduce the concentration(s) of toxic compounds to values below their respective TLV(s) such that the container 100 can be entered safely by workers. In the example of Fig. 1, the processor 113 determines that 2 hours 18 minutes is the advised aeration time for container 100. The highest exceedance factor determines the aeration time, with a minimum of 30 minutes when the highest exceedance factor is equal to 1. Thereto, the exceedance factor of every compound is calculated by dividing the measured concentration of that compound by its respective TLV. The compound with the highest exceedance factor is then selected as the critical compound that will determine the aeration time. In the above example this is benzene as benzene is detected in a concentration that exceeds its TLV by a factor of 4.6. The aeration time can then be calculated by multiplying the highest exceedance factor with a predetermined time interval, e.g. 30 minutes. It is noticed that in this example, other compounds do not influence the aeration time as substances are supposed to air proportionally. Consequently, the compound with the highest exceedance factor may be used to determine the aeration time. Thus, although 1,2-dichloroethane is detected in a concentration that exceeds its TLV, its exceedance factor of 1.46 is below the exceedance factor 4.6 of benzene and therefore its concentration is assumed to have dropped below its TLV as a result of the aeration for an aeration time that is calculated from the higher exceedance factor. Parallel to the air sample analysis performed by the air sample analyser 111, the expert technician 110 uses a generic VOC sensor 112, a PID 10.6eV lamp calibrated with isobutylene, to obtain a VOC reading for the air sample extracted from container 100. The VOC reading is communicated to the processor 113 which in turn calculates a safe VOC reading from the risk inventory and the VOC reading received from sensor 112. In the example of Fig. 1, the processor 113 determines that 7.1 ppm is a safe VOC reading for container 100. The VOC concentration, measured during the first analysis with the generic sensor, is determined as 32.9 ppm. Using this value and dividing it by the highest exceedance factor calculated here above, i.e. 4.6, a safe VOC reading of 7.1 ppm is established. It is noticed that it is of no importance whether the critical compound, i.e. benzene in the above example, contributes to the generic VOC reading or not. Processor 113 thereupon produces a risk evaluation report 114 for container 100. This risk evaluation report 114 contains at least the recommended aeration time of 2 hours 18 minutes 141 and the safe VOC reading 142 of 7.1 ppm for container 100. The risk evaluation report 114 may for instance be a printed document that will accompany container 100 when the latter is transported by truck 115 to a decentralized location 105. At quay 101, the air sample analyser 111, VOC sensor 112 and processor 113 are used to produce a similar risk evaluation report for all containers 100, 104 unshipped there.

It is noticed that in the above example, the aeration time is directly proportional to the exceedance factor of the critical compound. In alternative embodiments however, more complex formulas may be applied to determine the recommended aeration time from the exceedance factors. These formulas may be non-linear, for instance for compounds that are water soluble and therefore reduce more slowly or non-linearly in concentration during aeration. These formulas may also consider plural compounds, if for instance it is known that presence of one compound has an effect on the concentration reduction of another compound during aeration. Also the calculation of the safe generic VOC reading may be more complex, i.e. depending on plural compounds and not necessarily a linear function of the measured concentrations or exceedance factors.

Upon arrival at the decentralized location 105, container 100 is aired for the minimum duration time 141 mentioned in the risk evaluation report 114 accompanying container 100. Thereafter, a person 116 who does not need to be an expert technician uses a VOC sensor 117 to obtain a VOC sensor reading for a second air sample of the container 100. The VOC sensor reading is compared with the safe VOC value 142 mentioned in the risk evaluation report 114 accompanying container 100. Only when the VOC sensor reading is below the safe VOC value 142, the container 100 shall be released for unloading or physical inspection at the decentralized location 105. When the VOC sensor reading is above the safe VOC value 142, the container 100 will be aired for a supplementary aeration time, for instance a predetermined minimum time interval. This process may be repeated iteratively until the person 116 obtains a VOC sensor reading that is below the safe VOC value 142 mentioned in the risk evaluation report 114.

At the decentralized location 105, no complete air sample analysis by a bulky, expensive air sample analyser such as 111 is required. A simple VOC sensor reading is sufficient to enable even non-technical personnel to declare the container 100 safe for unloading or physical inspection. This also holds true at any time during the inspection or unloading process.

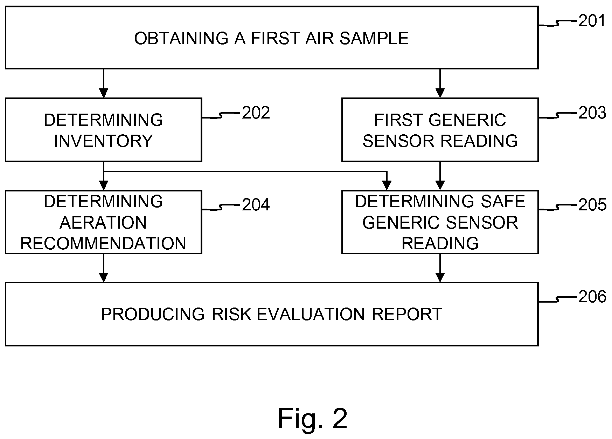

Fig. 2 shows the steps executed in an embodiment of the method according to the invention at the centralized location, e.g. quay 101 in Fig. 1. In a first step 201, a first air sample is obtained from a container 100 that has been unshipped. In a second step 202, an air sample analyser determines an inventory of compounds present in the first air sample and the respective concentrations of these compounds in the first air sample. In a parallel third step 203, a generic sensor is used to obtain a first generic sensor reading for the first air sample. The inventory of compounds and concentrations is used in a fourth step 204 to determine a recommended aeration time for the container 100. This recommended aeration time may be extracted from tables or may be calculated through formulas making use of the concentrations of potentially toxic compounds detected in the first air sample and knowledge on safe values for the concentrations of these compounds. The risk inventory as established by the air sample analyser and the first generic sensor reading are further used in a fifth step 205 to determine a safe generic sensor reading. The safe generic sensor reading may for instance be obtained through linear regression if it is assumed that the generic sensor value decreases proportionally with the concentration of the toxic compound that exceeds its TLV most. Alternative formula however may be applied if a toxic compound has been detected whose concentration reduces more slowly, for instance a dangerous gas that is soluble in water and therefore reduces more slowly in concentration during aeration. The recommended aeration time as determined in step 204 and the safe generic sensor value as determined in step 205 are mentioned in a risk evaluation report for container 100 that is generated in step 206. The risk evaluation report may optionally contain additional information like for instance the list of compounds in the first air sample that exceed their TLV(s), and the measured concentrations of these compounds, although this information is no longer mandatory in order to enable verification and release of the container for unloading or physical inspection at a remote, decentralized location.

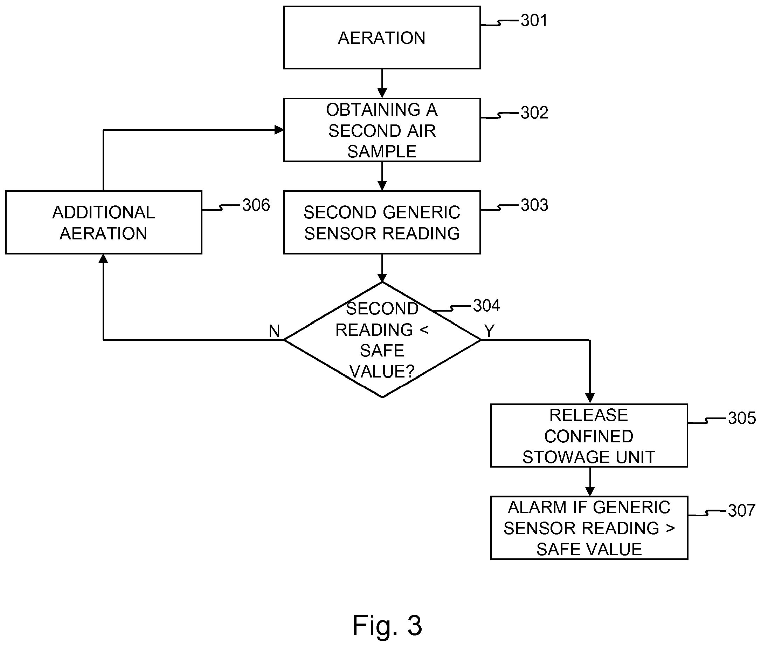

Fig. 3 shows the steps executed in an embodiment of the present invention at the decentralized location where the container 100 is transported to before it is opened, for instance 105 in Fig. 1. In a first step 301, the container 100 is aired in line with the aeration recommendation mentioned in the risk inventory report accompanying the container 100. The container 100 thereto may be brought to a separate terrain where it is aired for a time interval at least equal to the aeration time 141 mentioned in the risk inventory report 114. Thereafter, in a second step 302, a second air sample is obtained from the container 100. In a third step 303, a generic sensor is used to obtain a generic sensor reading for the second air sample. In step 304, this generic sensor reading is then compared with the safe generic sensor value 142 mentioned in the risk inventory report 114 accompanying container 100. If the generic sensor reading is below the safe generic sensor value, the container 100 is released for unloading or physical inspection in step 305. In case the generic sensor reading is on or above the safe generic sensor value, the container 100 is returned to the separate terrain for additional aeration in step 306. Subsequently, the steps 302, 303, 304 and 306 are reiterated until an air sample is obtained from the container 100 with generic sensor reading below the safe generic sensor value 142. After the container 100 has been released for unloading or physical inspection in step 305, the gas concentrations may be continuously monitored through iterative generic sensor measurements. As soon as a generic sensor measurement exceeds the safe generic sensor value an alarm is generated in step 307, for instance an audible or visible alarm. This way, one can guarantee that workers involved in the unloading or inspection process can operate in permanent safe working conditions.

At the decentralized location 105 where the container 100 is verified before release, no specialized personnel is required and no expensive equipment must be transported to this location for the verification process. A simple generic sensor reading obtained by a generic sensor that can be operated by any non-technician, is sufficient to verify and safely release the container 100 for unloading or physical inspection.

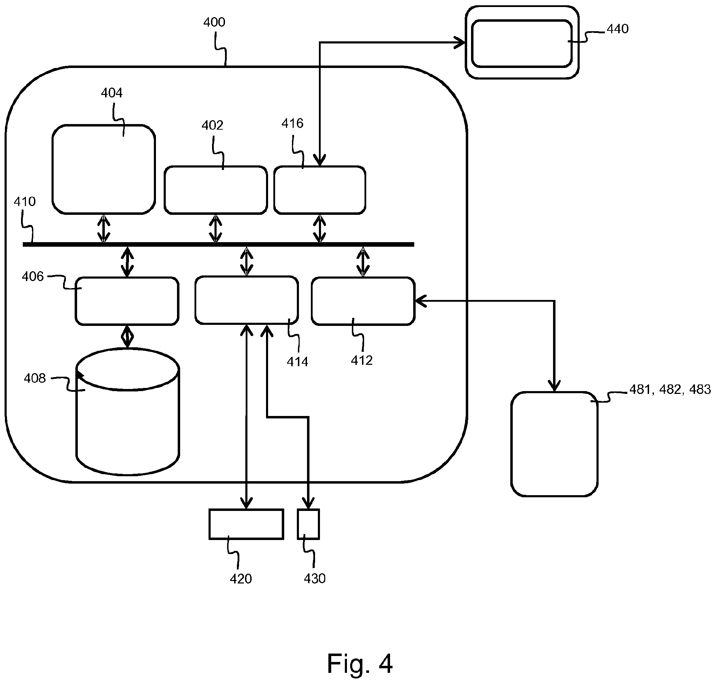

Fig. 4 shows a suitable computing system or processing system 400 as used in embodiments of the invention. Computing system 400 is suitable for implementing unit 113 in the above described embodiment. Computing system 400 may in general be formed as a suitable general-purpose computer and comprise a bus 410, a processor 402, a local memory 404, one or more optional input interfaces 414, one or more optional output interfaces 416, a communication interface 412, a storage element interface 406 and one or more storage elements 408. Bus 410 may comprise one or more conductors that permit communication among the components of the computing system 400. Processor 402 may include any type of conventional processor or microprocessor that interprets and executes programming instructions. Local memory 404 may include a random access memory (RAM) or another type of dynamic storage device that stores information and instructions for execution by processor 402 and/or a read only memory (ROM) or another type of static storage device that stores static information and instructions for use by processor 402. Input interface 414 may comprise one or more conventional mechanisms that permit an operator to input information to the computing device 400, such as a keyboard 420, a mouse 430, a pen, voice recognition and/or biometric mechanisms, etc. Output interface 416 may comprise one or more conventional mechanisms that output information to the operator, such as a display 440, etc. Communication interface 412 may comprise any transceiver-like mechanism such as for example one or more Ethernet interfaces that enables computing system 400 to communicate with other devices and/or systems, for example with other computing devices 481, 482, 483. The communication interface 412 of computing system 400 may be connected to such another computing system by means of a local area network (LAN) or a wide area network (WAN) such as for example the internet. Storage element interface 406 may comprise a storage interface such as for example a Serial Advanced Technology Attachment (SATA) interface or a Small Computer System Interface (SCSI) for connecting bus 410 to one or more storage elements 408, such as one or more local disks, for example SATA disk drives, and control the reading and writing of data to and/or from these storage elements 408. Although the storage elements 408 above is described as a local disk, in general any other suitable computer-readable media such as a removable magnetic disk, optical storage media such as a CD or DVD, -ROM disk, solid state drives, flash memory cards, ... could be used. It is noticed that the entire method according to the present invention can be executed in a management centre or on the target WLAN access point. Computing system 400 could thus correspond to the processing system 113 when reflecting back to Fig. 1.

## Claims

1. A method to release a confined stowage unit (100) for unloading or physical inspection, said method comprising:
A. an air sample analyser (111) at a central location (101) with multiple confined stowage units (100, 104) analysing a first air sample of said confined stowage unit (100) and determining an inventory of gases present in said first air sample with their gas concentrations;
B. a first generic sensor (112) measuring at said central location (101) a first generic sensor value for said first air sample, a generic sensor being a sensor providing a read-out value, named the generic sensor value, that is indicative for the presence of one or several compounds that are not necessarily dangerous, and that can be related to the concentration or evolution of the concentration of one or several toxic compounds in said inventory;
**characterised by**
C. a processor (113) determining a safe generic sensor value (142) from said gas concentrations in said inventory and said first generic sensor value, and producing a risk evaluation report (114) for said confined stowage unit (100), said risk evaluation report (114) comprising an aeration recommendation (141) and said safe generic sensor value (142);
D. a second generic sensor (117) similar to said first generic sensor (112) measuring at a decentralized location (105) whereto said confined stowage unit (100) is transported and where said confined stowage unit (100) has been aired according to said aeration recommendation (141), a second generic sensor value for a second air sample of said confined stowage unit (100);
E. comparing said second generic sensor value with said safe generic sensor value (142); and
F. releasing said confined stowage unit (100) for unloading or physical inspection if said second generic sensor value is below said safe generic sensor value (142).

2. The method according to claim 1, further comprising iteratively repeating said steps D and E until said second generic sensor value is below said safe generic sensor value (142) and supplementary airing said confined stowage unit for a predetermined time between subsequent iterations of said steps D and E as long as said second generic sensor value is above said safe generic sensor value (142).

3. The method according to claim 1, further comprising iteratively repeating said steps D and E at any time during unloading or physical inspection for the purpose of monitoring said gas concentrations and evaluating the safety of workers involved in said unloading or physical inspection.

4. The method according to claim 3, further comprising generating a visual or audible alarm if said second generic sensor value is above said safe generic sensor value (142).

5. The method according to claim 1, wherein said first generic sensor (112) and said second generic sensor (117) are VOC sensors or photoionization detectors.

6. The method according to claim 1, wherein said first generic sensor and said second generic sensor are electrochemical sensors.

7. The method according to claim 1, wherein said first generic sensor and said second generic sensor are diode/heated pentode sensors.

8. The method according to claim 1, wherein said first generic sensor and said second generic sensor are infrared sensors.

9. The method according to claim 1, wherein said first generic sensor and said second generic sensor are catalytic sensors.

10. The method according to claim 1, wherein at said decentralized location (105) no further analysis of said second air sample is executed except said second generic sensor value measurement.

11. The method according to claim 1, wherein said risk evaluation report comprises a machine-readable code wherein said aeration recommendation (141) and/or said safe generic sensor value (142) are encoded.

12. A system for releasing a confined stowage unit (100) for unloading or physical inspection, said system comprising:
A. an air sample analyser (111) adapted to analyse a first air sample of said confined stowage unit (100) at a central location (101) with multiple confined stowage units (100, 104), and configured to determine an inventory of gases present in said first air sample with their gas concentrations;
B. a first generic sensor (112) adapted to measure at said central location (101) a first generic sensor value for said first air sample, a generic sensor being a sensor providing a read-out value, named the generic sensor value, that is indicative for the presence of one or several compounds that are not necessarily dangerous, and that can be related to the concentration or evolution of the concentration of one or several toxic compounds in said inventory;
**characterised by**
C. a processor (113) configured to determine a safe generic sensor value (142) from said gas concentrations in said inventory and said first generic sensor value, and configured to produce a risk evaluation report (114) for said confined stowage unit (100), said risk evaluation report (114) comprising an aeration recommendation (141) and said safe generic sensor value (142);
D. a second generic sensor (117) similar to said first generic sensor (112), adapted to measure at a decentralized location (105) whereto said confined stowage unit (100) is transported and where said confined stowage unit (100) has been aired according to said airing recommendation (141), a second generic sensor value for a second air sample of said confined stowage unit (100) for comparison with said safe generic sensor value (142), and release of said confined stowage unit (100) for unloading or physical inspection if said second generic sensor value is below said safe generic sensor value (142).

13. The system for releasing a confined stowage unit (100) according to claim 12, wherein:
- said processor (113) is configured to generate a machine-readable code wherein said aeration recommendation (141) and/or said safe generic sensor value (142) are encoded, and to embed said machine-readable code in said risk evaluation report (114); and
- said second generic sensor (117) comprises or connects with a code reader adapted to read said machine-readable code and to extract therefrom said aeration recommendation (141) and/or said safe generic sensor value (142).

14. The system for releasing a confined stowage unit (100) according to claim 12 or claim 13, wherein:
- said second generic sensor (117) comprises an alarm unit configured to generate a visual or audible alarm if said second generic sensor value is above said safe generic sensor value (142).

## Patentansprüche

1. Verfahren zum Freigeben einer geschlossenen Laderaumeinheit (100) zum Entladen oder zur physischen Inspektion, das Verfahren umfassend:
A. ein Luftprobenanalysegerät (111) an einer zentralen Stelle (101) mit vielfachen geschlossenen Laderaumeinheiten (100, 104), das eine erste Luftprobe der eingeschlossenen Laderaumeinheit (100) analysiert und ein Inventar von Gasen, die in der ersten Luftprobe vorhandenen sind, mit ihren Gaskonzentrationen bestimmt;
B. einen ersten generischen Sensor (112), der an der zentralen Stelle (101) einen ersten generischen Sensorwert für die erste Luftprobe misst, wobei ein generischer Sensor ein Sensor ist, der einen Auslesewert bereitstellt, der als der generische Sensorwert bezeichnet wird, der indikativ für das Vorhandensein einer oder mehrerer Verbindungen ist, die nicht unbedingt gefährlich sind, und der mit der Konzentration oder Entwicklung der Konzentration von einer oder mehreren toxischen Verbindungen in dem Inventar in Verbindung gebracht werden kann;
**gekennzeichnet durch**
C. einen Prozessor (113), der aus den Gaskonzentrationen in dem Inventar und dem ersten generischen Sensorwert einen sicheren generischen Sensorwert (142) bestimmt und einen Risikobewertungsbericht (114) für die geschlossene Laderaumeinheit (100) erstellt, wobei der Risikobewertungsbericht (114) eine Lüftungsempfehlung (141) und den sicheren generischen Sensorwert (142) umfasst;
D. einen zweiten generischen Sensor (117) ähnlich wie der erste generische Sensor (112), der an einer dezentralisierten Stelle (105), an die die geschlossene Laderaumeinheit (100) transportiert wird und wo die geschlossene Laderaumeinheit (100) gemäß der Lüftungsempfehlung (141) gelüftet worden ist, einen zweiten generischen Sensorwert für eine zweite Luftprobe der geschlossenen Laderaumeinheit (100) misst;
E. Vergleichen des zweiten generischen Sensorwerts mit dem sicheren generischen Sensorwert (142); und
F. Freigeben der geschlossenen Laderaumeinheit (100) zum Entladen oder zur physischen Inspektion, wenn der zweite generische Sensorwert unter dem sicheren generischen Sensorwert (142) ist.

2. Verfahren nach Anspruch 1, ferner umfassend ein iteratives Wiederholen der Schritte D und E, bis der zweite generische Sensorwert unter dem sicheren generischen Sensorwert (142) ist , und zusätzliches Lüften der begrenzten Laderaumeinheit über eine vorbestimmte Zeit zwischen aufeinanderfolgenden Iterationen der Schritte D und E, solange der zweite generische Sensorwert über dem sicheren generischen Sensorwert (142) ist.

3. Verfahren nach Anspruch 1, ferner ein iteratives Wiederholen der Schritte D und E jederzeit während eines Entladens oder einer physischen Inspektion zum Zweck einer Überwachung der Gaskonzentrationen und Bewertung der Sicherheit der Arbeiter, die an dem Entladen oder der physischen Inspektion involviert sind.

4. Verfahren nach Anspruch 3, ferner umfassend ein Erzeugen eines visuellen oder akustischen Alarms, wenn der zweite generische Sensorwert über dem sicheren generischen Sensorwert (142) ist.

5. Verfahren nach Anspruch 1, wobei der erste generische Sensor (112) und der zweite generische Sensor (117) VOC-Sensoren oder Photoionisationsdetektoren sind.

6. Verfahren nach Anspruch 1, wobei der erste generische Sensor und der zweite generische Sensor elektrochemische Sensoren sind.

7. Verfahren nach Anspruch 1, wobei der erste generische Sensor und der zweite generische Sensor Dioden/beheizte Pentode-Sensoren sind.

8. Verfahren nach Anspruch 1, wobei der erste generische Sensor und der zweite generische Sensor Infrarotsensoren sind.

9. Verfahren nach Anspruch 1, wobei der erste generische Sensor und der zweite generische Sensor katalytische Sensoren sind.

10. Verfahren nach Anspruch 1, wobei an der dezentralisierten Stelle (105) keine weitere Analyse der zweiten Luftprobe mit Ausnahme der zweiten generischen Sensorwertmessung ausgeführt wird.

11. Verfahren nach Anspruch 1, wobei der Risikobewertungsbericht maschinenlesbaren Code umfasst, in dem die Lüftungsempfehlung (141) und/oder der sichere generische Sensorwert (142) kodiert sind.

12. System zum Freigeben einer geschlossenen Laderaumeinheit (100) zum Entladen oder zur physischen Inspektion, das System umfassend:
A. einen Luftprobenanalysegerät (111), das angepasst ist, um eine erste Luftprobe der geschlossenen Laderaumeinheit (100) an einer zentralen Stelle (101) mit vielfachen geschlossenen Laderaumeinheiten (100, 104) zu analysieren, und das konfiguriert ist, um ein Inventar der in der ersten Luftprobe vorhandenen Gase mit ihren Gaskonzentrationen zu bestimmen;
B. einen ersten generischen Sensor (112), der angepasst ist, an der zentralen Stelle (101) einen ersten generischen Sensorwert für die erste Luftprobe zu messen, wobei ein generischer Sensor ein Sensor ist, der einen Auslesewert bereitstellt, der als der generische Sensorwert bezeichnet wird, der indikativ für das Vorhandensein einer oder mehrerer Verbindungen hinweist, die nicht unbedingt gefährlich sind, und der mit der Konzentration oder Entwicklung der Konzentration von einer oder mehreren toxischen Verbindungen in dem Inventar in Verbindung gebracht werden kann;
**gekennzeichnet durch**
C. einen Prozessor (113), der konfiguriert ist, um aus den Gaskonzentrationen in dem Inventar und dem ersten generischen Sensorwert einen sicheren generischen Sensorwert (142) zu bestimmen, und konfiguriert ist, um einen Risikobewertungsbericht (114) für die geschlossene Laderaumeinheit (100) zu erstellen, wobei der Risikobewertungsbericht (114) eine Lüftungsempfehlung (141) und den sicheren generischen Sensorwert (142) umfasst;
D. einen zweiten generischen Sensor (117) ähnlich wie der erste generische Sensor (112), der angepasst ist, um an einer dezentralisierten Stelle (105), an die die geschlossene Laderaumeinheit (100) transportiert wird und wo die geschlossene Laderaumeinheit (100) gemäß der Lüftungsempfehlung (141) gelüftet worden ist, einen zweiten generischen Sensorwert für eine zweite Luftprobe der geschlossenen Laderaumeinheit (100) zum Vergleich mit dem sicheren generischen Sensorwert (142) zu messen, und Freigeben der geschlossenen Laderaumeinheit (100) zum Entladen oder zur physischen Inspektion, wenn der zweite generische Sensorwert unter dem sicheren generischen Sensorwert (142) ist.

13. System zum Freigeben einer geschlossenen Laderaumeinheit (100) nach Anspruch 12, wobei:
- der Prozessor (113) konfiguriert ist, um einen maschinenlesbaren Code zu erzeugen, in dem die Lüftungsempfehlung (141) und/oder der sichere generische Sensorwert (142) codiert sind, und um den maschinenlesbaren Code in den Risikobewertungsbericht (114) einzubetten; und
- der zweite generische Sensor (117) ein Codelesegerät umfasst oder damit verbunden ist, das angepasst ist, um den maschinenlesbaren Code zu lesen und daraus die Lüftungsempfehlung (141) und/oder den sicheren generischen Sensorwert (142) zu extrahieren.

14. System zum Freigeben einer geschlossenen Laderaumeinheit (100) nach Anspruch 12 oder Anspruch 13, wobei:
- der zweite generische Sensor (117) eine Alarmeinheit umfasst, die konfiguriert ist, um einen visuellen oder akustischen Alarm zu erzeugen, wenn der zweite generische Sensorwert über dem sicheren generischen Sensorwert (142) liegt.

## Revendications

1. Procédé pour libérer une unité de cargaison fermée (100) en vue du déchargement ou d'une inspection physique, ledit procédé comprenant :
A. un analyseur d'échantillon d'air (111) au niveau d'un emplacement central (101) avec de multiples unités de cargaison fermées (100, 104) analysant un premier échantillon d'air de ladite unité de cargaison fermée (100) et déterminant un inventaire de gaz présents dans ledit premier échantillon d'air avec leurs concentrations de gaz ;
B. un premier capteur générique (112) mesurant au niveau dudit emplacement central (101) une première valeur de capteur générique pour ledit premier échantillon d'air, un capteur générique étant un capteur fournissant une valeur de lecture, appelée la valeur de capteur générique, qui est indicative de la présence d'un ou plusieurs composés qui ne sont pas nécessairement dangereux, et qui peuvent être liés à la concentration ou à l'évolution de la concentration d'un ou plusieurs composés toxiques dans ledit inventaire ;
**caractérisé par**
C. un processeur (113) déterminant une valeur de capteur générique sûre (142) à partir desdites concentrations de gaz dans ledit inventaire et de ladite première valeur de capteur générique, et produisant un rapport d'évaluation des risques (114) pour ladite unité de cargaison fermée (100), ledit rapport d'évaluation des risques (114) comprenant une recommandation d'aération (141) et ladite valeur de capteur générique sûre (142) ;
D. un second capteur générique (117) similaire audit premier capteur générique (112) mesurant au niveau d'un emplacement décentralisé (105) vers lequel ladite unité de cargaison fermée (100) est transportée et où ladite unité de cargaison fermée (100) a été aérée selon ladite recommandation d'aération (141), une seconde valeur de capteur générique pour un second échantillon d'air de ladite unité de cargaison fermée (100) ;
E. la comparaison de ladite seconde valeur de capteur générique avec ladite valeur de capteur générique sûre (142) ; et
F. la libération de ladite unité de cargaison fermée (100) en vue d'un déchargement ou d'une inspection physique si ladite seconde valeur de capteur générique est inférieure à ladite valeur de capteur générique sûre (142).

2. Procédé selon la revendication 1, comprenant en outre la répétition itérative desdites étapes D et E jusqu'à ce que ladite seconde valeur de capteur générique soit inférieure à ladite valeur de capteur générique sûre (142) et l'aération supplémentaire de ladite unité de cargaison fermée pendant une durée prédéfinie entre les itérations suivantes desdites étapes D et E tant que ladite seconde valeur de capteur générique est supérieure à ladite valeur de capteur générique sûre (142).

3. Procédé selon la revendication 1, comprenant en outre la répétition itérative desdites étapes D et E à tout moment durant le déchargement ou l'inspection physique dans le but de surveiller lesdites concentrations de gaz et d'évaluer la sécurité des travailleurs impliqués dans ledit déchargement ou ladite inspection physique.

4. Procédé selon la revendication 3, comprenant en outre la génération d'une alarme visuelle ou sonore si ladite seconde valeur de capteur générique est supérieure à ladite valeur de capteur générique sûre (142).

5. Procédé selon la revendication 1, ledit premier capteur générique (112) et ledit second capteur générique (117) étant des capteurs de composés organiques volatiles (COV) ou des détecteurs de photoionisation.

6. Procédé selon la revendication 1, ledit premier capteur générique et ledit second capteur générique étant des capteurs électrochimiques.

7. Procédé selon la revendication 1, ledit premier capteur générique et ledit second capteur générique étant des capteurs à diode/pentode chauffée.

8. Procédé selon la revendication 1, ledit premier capteur générique et ledit second capteur générique étant des capteurs infrarouges.

9. Procédé selon la revendication 1, ledit premier capteur générique et ledit second capteur générique étant des capteurs catalytiques.

10. Procédé selon la revendication 1, au niveau dudit emplacement décentralisé (105), aucune analyse supplémentaire dudit second échantillon d'air n'est exécutée à l'exception de ladite seconde mesure de valeur de capteur générique.

11. Procédé selon la revendication 1, ledit rapport d'évaluation des risques comprenant un code lisible par machine dans lequel ladite recommandation d'aération (141) et/ou ladite valeur de capteur générique sûre (142) sont codées.

12. Système destiné à libérer une unité de cargaison fermée (100) en vue du déchargement ou d'une inspection physique, ledit système comprenant :
A. un analyseur d'échantillon d'air (111) adapté pour analyser un premier échantillon d'air de ladite unité de cargaison fermée (100) au niveau d'un emplacement central (101) avec de multiples unités de cargaison fermées(100, 104), et configuré pour déterminer un inventaire des gaz présents dans ledit premier échantillon d'air avec leurs concentrations de gaz ;
B. un premier capteur générique (112) adapté pour mesurer au niveau dudit emplacement central (101) une première valeur de capteur générique pour ledit premier échantillon d'air, un capteur générique étant un capteur fournissant une valeur de lecture, appelée valeur de capteur générique, qui est indicative de la présence d'un ou plusieurs composés qui ne sont pas nécessairement dangereux, et qui peuvent être liés à la concentration ou à l'évolution de la concentration d'un ou plusieurs composés toxiques dans ledit inventaire ;
**caractérisé par**
C. un processeur (113) configuré pour déterminer une valeur de capteur générique sûre (142) à partir desdites concentrations de gaz dans ledit inventaire et de ladite première valeur de capteur générique, et configuré pour produire un rapport d'évaluation des risques (114) pour ladite unité de cargaison fermée (100), ledit rapport d'évaluation des risques (114) comprenant une recommandation d'aération (141) et ladite valeur de capteur générique sûre (142) ;
D. un second capteur générique (117) similaire audit premier capteur générique (112), adapté pour mesurer, au niveau d'un emplacement décentralisé (105) vers lequel ladite unité de cargaison fermée (100) est transportée et où ladite unité de cargaison fermée (100) a été aérée selon ladite recommandation d'aération (141), une seconde valeur de capteur générique pour un second échantillon d'air de ladite unité de cargaison fermée (100) pour comparaison avec ladite valeur de capteur générique sûre (142), et la libération de ladite unité de cargaison fermée (100) en vue du déchargement ou d'une inspection physique si ladite seconde valeur de capteur générique est inférieure à ladite valeur de capteur générique sûre (142).

13. Système destiné à libérer une unité de cargaison fermée (100) selon la revendication 12,
- ledit processeur (113) étant configuré pour générer un code lisible par machine, ladite recommandation d'aération (141) et/ou ladite valeur de capteur générique sûre (142) étant codées, et pour incorporer ledit code lisible par machine dans ledit rapport d'évaluation des risques (114) ; et
- ledit second capteur générique (117) comprenant ou se connectant à un lecteur de code adapté pour lire ledit code lisible par machine et en extraire ladite recommandation d'aération (141) et/ou ladite valeur de capteur générique sûre (142).

14. Système destiné à libérer une unité de cargaison fermée (100) selon la revendication 12 ou 13,
- ledit second capteur générique (117) comprenant une unité d'alarme configurée pour générer une alarme visuelle ou sonore si ladite seconde valeur de capteur générique est supérieure à ladite valeur de capteur générique sûre (142).
